# EUROPEAN PATENT APPLICATION

(11) **EP 4 424 356 A1**
(43) Date of publication of application: **04.09.2024**
(21) Application number: 22885894.0
(22) Date of filing: 25.10.2022
(51) Int. Cl.: A61M 60/00

(54) **PUMP BLOOD FLOW DETERMINATION METHOD AND APPARATUS FOR BLOOD PUMP, ELECTRONIC DEVICE, AND STORAGE MEDIUM**

(30) Priority: 25.10.2021 CN 202111243412
(71) Applicant: Forqaly Medical (Shanghai) Co., Ltd., Shanghai 201318 (CN)
(72) Inventor: LIU, Zhiyong, Shanghai 201318 (CN); XUE, Zhikuan, Shanghai 201318 (CN); CAO, Dianjia, Shanghai 201318 (CN)
(74) Representative: Barker Brettell LLP
(86) International application number: PCT/CN2022/127202
(87) International publication number: WO 2023/072017

(57) **Abstract**

A pump blood flow determination method and apparatus for a blood pump, an electronic device, and a storage medium. The method comprises: (S110) obtaining a first correspondence between absolute values of first current differences and first pump blood flow values at different rotational speeds of the blood pump in an in vitro test simulation environment; (S 120) obtaining first reference current values of current cycles at different rotational speeds of the blood pump in an actual human body environment of a test object; (S130) for the current cycles at the different rotational speeds, calculating differences between the first reference current values and current values at different moments within the current cycles, so as to obtain an absolute value of at least one second current difference; (S140) for the absolute values of the second current differences corresponding to the current cycles at the different rotational speeds, searching in the first correspondence to obtain second pump blood flow values corresponding to the absolute values of the second current differences, wherein the absolute value of each second current difference corresponds to a second pump blood flow value; and (S150) for each rotational speed, determining a target pump blood flow value on the basis of the second pump blood flow values in the current cycles.

## Description

### CROSS-REFERENCE TO RELATED APPLICATIONS

This application claims priority to Chinese Patent Application No. CN202111243412.4, entitled "METHOD AND DEVICE FOR QUANTIFYING PUMP BLOOD SUPPORT FLOW OF BLOOD PUMP", filed on October 25, 2021, which is hereby incorporated by reference in its entirety.

### TECHNICAL FIELD

The present application relates to the field of information processing, and in particular, to a pump blood flow determination method and apparatus for a blood pump, an electronic device, and a storage medium.

### BACKGROUND

A short-term blood pump is one of the effective ways to treat cardiovascular and related complications, which provides patients with hemodynamic support in a short term to promote rapid recovery of functions of the heart and other important tissues and organs. Effectiveness of the short-term blood pump is mainly reflected in improvement of a patient's aortic pressure and cardiac output. The patient's cardiac output supported by the short-term blood pump is mainly a sum of the patient's own cardiac output and pump blood flow of the blood pump. How to correctly calibrate the two indexes (the patient's own cardiac output and the pump blood flow of the blood pump) is particularly important.

At present, the pump blood flow is mainly calculated according to a relationship between a current of the blood pump and pump blood flow of a catheter, while the patient's own cardiac output is calculated on the basis of the pump blood flow. The applicant has found that differences between motors and catheters of different blood pumps may cause different currents in the blood pumps. Therefore, representation of the pump blood flow of the blood pump on the basis of the current in the blood pump is inaccurate.

### SUMMARY

One objective of embodiments of the present application is to provide a pump blood flow determination method and apparatus for a blood pump, an electronic device, and a storage medium to achieve an effect of accurately determining pump blood flow of the blood pump.

An embodiment of a first aspect of the present application provides a pump blood flow determination method for a blood pump, the method including:
obtaining a first correspondence between absolute values of first current differences and first pump blood flow values at different rotational speeds of the blood pump in an in vitro test simulation environment;
obtaining first reference current values of current cycles during operation of the blood pump at different rotational speeds in an actual human body environment of a test object;
for each current cycle at each rotational speed, calculating differences between the first reference current value and current values at different moments within the current cycle, so as to obtain an absolute value of at least one second current difference;
for the absolute value of the at least one second current difference corresponding to each current cycle at each rotational speed, searching in the first correspondence to obtain a second pump blood flow value corresponding to the absolute value of the second current difference, wherein the absolute value of each second current difference corresponds to a second pump blood flow value; and
for each rotational speed, determining a target pump blood flow value on the basis of at least one second pump blood flow value in each current cycle.

An embodiment of a second aspect of the present application provides a pump blood flow determination apparatus for a blood pump, the apparatus including:
a first obtaining module configured to obtain a first correspondence between absolute values of first current differences and first pump blood flow values at different rotational speeds of the blood pump in an in vitro test simulation environment;
a second obtaining module configured to obtain first reference current values of current cycles during operation of the blood pump at different rotational speeds in an actual human body environment of a test object;
a first determination module configured to, for each current cycle at each rotational speed, calculate differences between the first reference current value and current values at different moments within the current cycle, so as to obtain an absolute value of at least one second current difference;
a second determination module configured to, for the absolute value of the at least one second current difference corresponding to each current cycle at each rotational speed, search in the first correspondence to obtain at least one second pump blood flow value corresponding to the absolute value of the at least one second current difference; and
a third determination module configured to, for each rotational speed, determine a target pump blood flow value on the basis of at least one second pump blood flow value in each current cycle.

An embodiment of a third aspect of the present application provides an electronic device, including a processor, a memory, and programs or instructions stored in the memory and executable on the processor, wherein the programs or instructions, when executed by the processor, implement steps of the pump blood flow determination method for a blood pump according to any of the embodiments of the present application.

An embodiment of a fourth aspect of the present application provides a readable storage medium, wherein the readable storage medium stores programs or instructions, and the programs or instructions, when executed by a processor, implement steps of the pump blood flow determination method for a blood pump according to any of the embodiments of the present application.

The technical solutions provided in the embodiments of the present application bring at least the following beneficial effects.

According to the pump blood flow determination method for a blood pump provided in the embodiments of the present application, a first correspondence between absolute values of first current differences and first pump blood flow values at different rotational speeds of the blood pump in an in vitro test simulation environment is obtained; first reference current values of current cycles during operation of the blood pump at different rotational speeds in an actual environment of a test object are obtained; for each current cycle at each rotational speed, differences between the first reference current value and current values at different moments within the current cycle are calculated, so as to obtain an absolute value of at least one second current difference; for the absolute value of the at least one second current difference corresponding to each current cycle at each rotational speed, searching is performed in the first correspondence to obtain at least one second pump blood flow value corresponding to the absolute value of the at least one second current difference; and for each rotational speed, a target pump blood flow value is determined on the basis of at least one second pump blood flow value in each current cycle. Since current changes are mainly related to changes in pump blood flow values, compared with the manner of directly using currents to represent the pump blood flow values of the blood pump, in the manner of effectively representing the pump blood flow values of the blood pump with current change differences, by using the current differences, errors caused by differences between different blood pump catheters can be effectively reduced, thereby achieving an effect of accurately determining pump blood flow of the blood pump.

The general description above and the detailed description in the following are merely exemplary and illustrative, and cannot limit the present application.

### BRIEF DESCRIPTION OF THE DRAWINGS

The accompanying drawings herein are incorporated into the specification and form a part of this specification, illustrate embodiments that conform to the present application, are used for describing a principle of the present application together with this specification, and do not constitute any inappropriate limitation on the present application.
FIG. 1 is a schematic diagram of a pump blood flow determination method for a blood pump according to an exemplary embodiment of the present application;
FIG. 2 is a diagram of a second correspondence between pressure differences at different rotational speeds and second current values under the pressure differences according to an exemplary embodiment of the present application;
FIG. 3 is a diagram of a third correspondence between pressure differences at different rotational speeds and first pump blood flow values under the pressure differences according to an exemplary embodiment of the present application;
FIG. 4 is a diagram of a correspondence between pressure differences at different rotational speeds and absolute values of first current differences under the pressure differences according to an exemplary embodiment of the present application;
FIG. 5 is a diagram of a correspondence between absolute values of first current differences and first pump blood flow at different rotational speeds according to an exemplary embodiment of the present application;
FIG. 6 is a curve of current changes in a cardiac cycle at a rotational speed according to an exemplary embodiment of the present application;
FIG. 7 is a curve of second pump blood flow values in a current cycle at a rotational speed according to an exemplary embodiment of the present application;
FIG. 8 is a correction curve of absolute values of first current differences at a rotational speed n1 according to an exemplary embodiment of the present application;
FIG. 9 is a correction curve of absolute values of first current differences at a rotational speed n2 according to an exemplary embodiment of the present application;
FIG. 10 is a correction curve of absolute values of first current differences at a rotational speed n3 according to an exemplary embodiment of the present application;
FIG. 11 is a structural block diagram of a pump blood flow determination apparatus for a blood pump according to an exemplary embodiment of the present application; and
FIG. 12 is a schematic structural diagram of an electronic device according to an embodiment of the present application.

### DETAILED DESCRIPTION

In order to enable those of ordinary skill in the art to better understand the technical solutions of the present application, the technical solutions in the embodiments of the present application will be clearly and completely described below in conjunction with the accompanying drawings. It should be understood that specific embodiments described herein are only intended to explain the present application, but not to limit the present application. For those skilled in the art, the present application can be implemented without some of these specific details. The following description of the embodiments is merely to provide a better understanding of the present application by illustrating the examples of the present application.

It is to be noted that terms such as "first" and "second" used in the specification and claims of the present application and the above drawings are intended to distinguish similar objects, but are not necessarily intended to describe a specific sequence or precedence order. It should be understood that data used in this manner may be interchangeable where appropriate, so that the embodiments of the present application described herein may be realized in an order in addition to those illustrated or described herein. The implementations described in the following exemplary embodiments do not represent all implementations consistent with the present application. Instead, they are merely examples consistent with some aspects of the present application as detailed in the appended claims.

In order to facilitate understanding of the technical solutions of the present application, background of the embodiments of the present application is first introduced.

When a supply voltage of a motor of a blood pump remains constant, a current value reflects magnitude of output power of the motor. A larger current indicates that the motor does more external work. In a scheme of an in vitro pump blood motor, external work of the motor includes overcoming rotational friction work of an in vitro driven shaft rotor, friction work of a flexible drive shaft relative to a constraint layer, rotational friction work of in vivo bearings, rotational driving work of an impeller, a drive shaft, and bearings, and work done by the impeller on pump blood. Absolute values of currents of the motor reflect the work done by the impeller on pump blood, and also reflect a comprehensive effect of the above factors. When other factors remain unchanged, greater pump blood flow indicates that the impeller does more work on the blood and the current value is also greater. However, even catheters of a same model of blood pumps are different from one catheter to another. For example, due to fluctuations in tolerances between components, the friction work required to be overcome may be different. In use, different bending states of the catheter may also cause friction work of the drive shaft relative to the constraint layer to be different. In summary, the current values reflect total work done by the motor, which, as mentioned above, include power consumption of various factors. Therefore, it is inaccurate to use the current values of the motor to calculate work done by the corresponding impeller on the blood and then determine the pump blood flow.

In order to solve the above problem, an embodiment of the present application provides a pump blood flow determination method for a blood pump, in which a first correspondence between absolute values of first current differences and first pump blood flow values at different rotational speeds of the blood pump in an in vitro test simulation environment is obtained; first reference current values of current cycles during operation of the blood pump at different rotational speeds in an actual environment of a test object are obtained; for each current cycle at each rotational speed, differences between the first reference current value and current values at different moments within the current cycle are calculated, so as to obtain an absolute value of at least one second current difference; for the absolute value of the at least one second current difference corresponding to each current cycle at each rotational speed, searching is performed in the first correspondence to obtain at least one second pump blood flow value corresponding to the absolute value of the at least one second current difference; and for each rotational speed, a target pump blood flow value is determined on the basis of at least one second pump blood flow value in each current cycle. When a structure of the catheter is determined, other factors may not change (i.e., the rotational friction work of the in vitro driven shaft rotor, the friction work of the flexible drive shaft relative to the constraint layer, the rotational friction work of the in vivo bearings, and the rotational driving work of the impeller, the drive shaft, and the bearings are all constant). In this case, changes in current values are mainly caused by changes in the work done by the impeller on the blood. Therefore, the current differences can better reflect the changes in the pump blood flow values, and the pump blood flow values can be determined through absolute values of the current differences and a pump blood flow reference value. In this way, compared with the manner of directly using currents to represent the pump blood flow values of the blood pump, in the manner of effectively representing the pump blood flow values of the blood pump with the current change differences, by using the current differences, an effect of accurately determining pump blood flow can be achieved.

The pump blood flow determination method for a blood pump provided in the embodiments of the present application are described in detail below with reference to the accompanying drawings through specific embodiments and application scenarios.

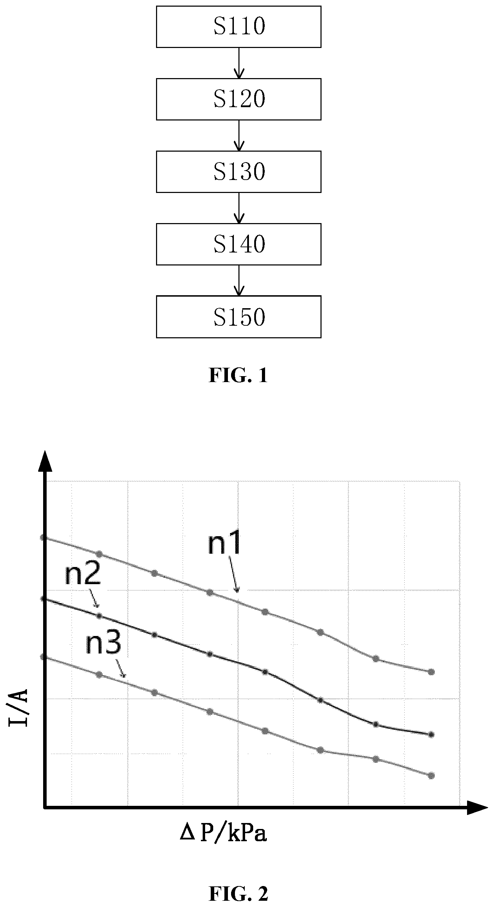

FIG. 1 is a schematic flowchart of a pump blood flow determination method for a blood pump according to an embodiment of the present application. As shown in FIG. 1, the pump blood flow determination method for a blood pump according to this embodiment of the present application may include the following steps.

In step S110, a first correspondence between absolute values of first current differences and first pump blood flow values at different rotational speeds of the blood pump in an in vitro test simulation environment is obtained.

In step S120, first reference current values of current cycles during operation of the blood pump at different rotational speeds in an actual human body environment of a test object is obtained.

In step S130, for each current cycle at each rotational speed, differences between the first reference current value and current values at different moments within the current cycle are calculated, so as to obtain an absolute value of at least one second current difference.

In step S140, for the absolute value of the at least one second current difference corresponding to each current cycle at each rotational speed, searching is performed in the first correspondence to obtain a second pump blood flow value corresponding to the absolute value of the second current difference. The absolute value of each second current difference corresponds to a second pump blood flow value.

In step S150, for each rotational speed, a target pump blood flow value is determined on the basis of at least one second pump blood flow value in each current cycle.

In this embodiment of the present application, a first correspondence between absolute values of first current differences and first pump blood flow values at different rotational speeds of the blood pump in an in vitro test simulation environment is obtained; first reference current values of current cycles during operation of the blood pump at different rotational speeds in an actual environment of a test object are obtained; for each current cycle at each rotational speed, differences between the first reference current value and current values at different moments within the current cycle are calculated, so as to obtain an absolute value of at least one second current difference; for the absolute value of the at least one second current difference corresponding to each current cycle at each rotational speed, searching is performed in the first correspondence to obtain at least one second pump blood flow value corresponding to the absolute value of the at least one second current difference; and for each rotational speed, a target pump blood flow value is determined on the basis of at least one second pump blood flow value in each current cycle. In this way, compared with the manner of directly using currents to represent the pump blood flow values of the blood pump, in the manner of effectively representing the pump blood flow values of the blood pump with current change differences, by using the current differences, errors caused by differences between different blood pump catheters can be effectively reduced, thereby achieving an effect of accurately determining pump blood flow of the blood pump.

The pump blood flow determination method for a blood pump provided in this embodiment of the present application will be described in detail below.

Firstly, step S110 of obtaining a first correspondence between absolute values of first current differences and first pump blood flow values at different rotational speeds of the blood pump in an in vitro test simulation environment is introduced.

The blood pump is an apparatus that provides patients with hemodynamic support.

In some embodiments of the present application, the in vitro test simulation environment may be an environment of a human body simulated in vitro. The in vitro test simulation environment is as close as possible to an environment inside the human body.

In an example, a fluid equal to or close to blood may be selected as a fluid in the in vitro test simulation environment, at a temperature equal to a blood temperature of a test object (i.e., the human body, which may be, for example, a patient) ranging from 36°C to 37°C. The fluid in the in vitro test simulation environment may be a mixture of water and glycerin at a ratio and with a viscosity equal to that of blood. The ratio may range from 45% to 55%.

In some embodiments of the present application, the absolute values of the first current differences may be absolute values of differences between a current value under a zero pressure difference and current values under other pressure differences obtained at different rotational speeds in the in vitro test simulation environment when a catheter of the blood pump is in a straight state. For example, in the in vitro test simulation environment, when the catheter of the blood pump is in the straight state, for a rotational speed n1, a current value I₀ under a zero pressure difference and current values I under other pressure differences at the rotational speed are obtained, differences between the current values I and I₀ under the pressure differences at the rotational speed n1 are calculated, and absolute values of the differences are calculated, so as to obtain absolute values of first current differences corresponding to the pressure differences.

The first pump blood flow values may be pump blood flow values under pressure differences at different rotational speeds.

The first correspondence may be a first correspondence between absolute values of first current differences and first pump blood flow values.

In some embodiments of the present application, in order to further accurately determine the target pump blood flow value of the blood pump, prior to step S110, the above pump blood flow determination method for a blood pump may further include:
obtaining a second correspondence between pressure differences and second current values and a third correspondence between the pressure differences and the first pump blood flow values of the blood pump at the different rotational speeds when the blood pump is in the in vitro test simulation environment;
for each rotational speed, taking the corresponding second current value under a zero pressure difference as a second reference current value;
for each rotational speed, obtaining second current values under different pressure differences at the rotational speed on the basis of the second correspondence, and calculating differences between the second reference current value and second current values under the different pressure differences at the rotational speed, so as to obtain absolute values of first current differences under the different pressure differences; and
for each rotational speed, obtaining the first correspondence between absolute values of first current differences and first pump blood flow values at the rotational speed on the basis of the absolute values of the first current differences and the third correspondence.

The second current values may be current values of the blood pump under different pressure differences in the catheter of the blood pump when the blood pump is in the in vitro test simulation environment. For example, when the blood pump is in the in vitro test simulation environment, corresponding current values of the blood pump under a zero pressure difference and other pressure differences in the catheter of the blood pump are obtained, so as to obtain the second current values corresponding to the pressure differences.

The second correspondence may be a correspondence between pressure differences and second current values under the pressure differences when the blood pump is in the in vitro test simulation environment and the blood pump is at the different rotational speeds. For example, when the blood pump is in the in vitro test simulation environment, for a rotational speed n1, a correspondence between pressure differences (including a zero pressure difference and other pressure differences) of the blood pump at the rotational speed n1 and second current values under the corresponding pressure differences is obtained, so as to obtain the second correspondence of the blood pump at the rotational speed n1. For a rotational speed n2, a correspondence between pressure differences (including a zero pressure difference and other pressure differences) of the blood pump at the rotational speed n2 and second current values under the corresponding pressure differences is obtained, so as to obtain the second correspondence of the blood pump at the rotational speed n2. For a rotational speed n3, a correspondence between pressure differences (including a zero pressure difference and other pressure differences) of the blood pump at the rotational speed n3 and second current values under the corresponding pressure differences is obtained, so as to obtain the second correspondence of the blood pump at the rotational speed n3.

The third correspondence may be a correspondence between pressure differences and first pump blood flow values under the pressure differences when the blood pump is in the in vitro test simulation environment and the blood pump is at the different rotational speeds. For example, when the blood pump is in the in vitro test simulation environment, for the rotational speed n1, a correspondence between pressure differences (including a zero pressure difference and other pressure differences) of the blood pump at the rotational speed n1 and first pump blood flow values under the corresponding pressure differences is obtained, so as to obtain the third correspondence of the blood pump at the rotational speed n1. For the rotational speed n2, a correspondence between pressure differences (including a zero pressure difference and other pressure differences) of the blood pump at the rotational speed n2 and first pump blood flow values under the corresponding pressure differences is obtained, so as to obtain a third correspondence of the blood pump at the rotational speed n2. For the rotational speed n3, a correspondence between pressure differences (including a zero pressure difference and other pressure differences) of the blood pump at the rotational speed n3 and first pump blood flow values under the corresponding pressure differences is obtained, so as to obtain the third correspondence of the blood pump at the rotational speed n3.

The second reference current value may be a current value of the catheter of the blood pump under the zero pressure difference when the blood pump is in the in vitro test simulation environment.

In some embodiments of the present application, the pressure difference may be a pressure difference between the heart and other organs in a normal human body. The pressure difference is in a range generally from 0 KPa to 14 KPa. In some embodiments of the present application, a pressure difference between a suction end and an outflow end of a pump blood catheter of the blood pump may be adjusted to the pressure difference between the heart and the other organs in the normal human body, so as to determine current values and pump blood flow values under different pressure differences through the blood pump.

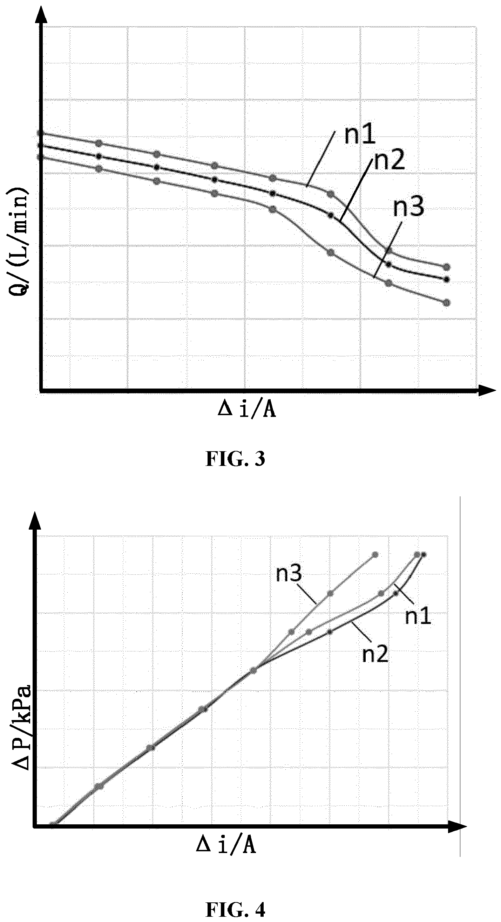

In an example, the blood pump may operate at different rotational speeds in the in vitro test simulation environment, second current values under different pressure differences and first pump blood flow values under the different pressure differences at the different rotational speeds are obtained, and then the second current values under the different pressure differences at the different rotational speeds are fit to obtain the second correspondence (i.e., FIG. 2), and the first pump blood flow values under the different pressure differences at the different rotational speeds are fit to obtain the third correspondence (i.e., FIG. 3).

In FIG. 2 and FIG. 3, n1, n2, and n3 respectively denote different rotational speeds.

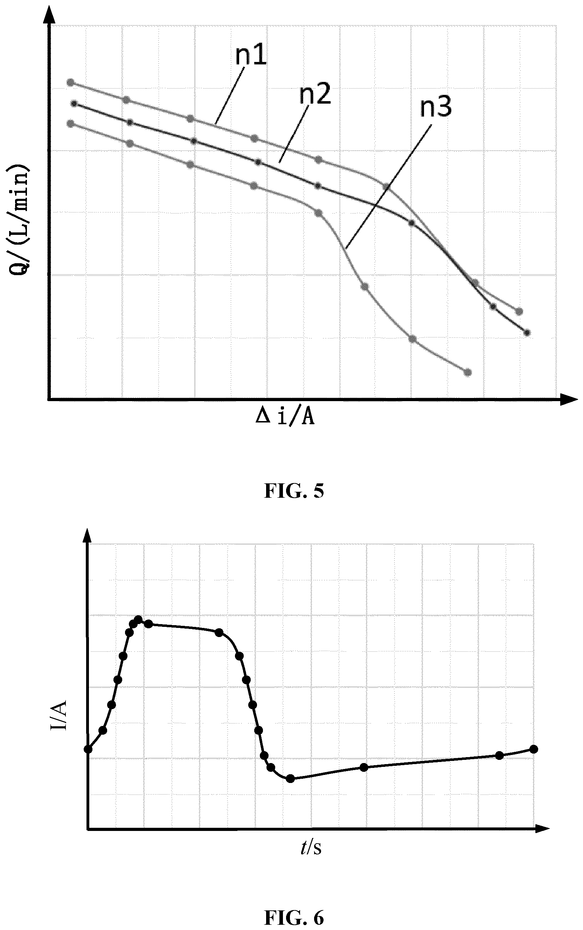

Still referring to the above example, after the second correspondence and the third correspondence are determined, a current value under a zero pressure difference at the different rotational speeds may be obtained and taken as the second reference current value. For example, for the rotational speed n1, a current value I₀ under the zero pressure difference at the rotational speed is obtained, and I₀ is taken as the second reference current value. Then, differences between the current values I and I₀ under the pressure differences at the rotational speed n1 are calculated according to the current values under the pressure differences in the second correspondence, and absolute values of the differences are calculated, so as to obtain absolute values of first current differences corresponding to the pressure differences (i.e., FIG. 4). Then, a correspondence between the absolute values of the first current differences and the first pump blood flow (i.e., FIG. 5) may be obtained according to the absolute values of the first current differences and the third correspondence.

In this embodiment of the present application, a second correspondence between pressure differences and second current values and a third correspondence between the pressure differences and the first pump blood flow values of the blood pump at the different rotational speeds are obtained when the blood pump is in the in vitro test simulation environment; for each rotational speed, the corresponding second current value under a zero pressure difference is taken as a second reference current value; for each rotational speed, second current values under different pressure differences at the rotational speed are obtained on the basis of the second correspondence, and differences between the second reference current value and second current values under the different pressure differences at the rotational speed are calculated, so as to obtain absolute values of first current differences under the different pressure differences; and for each rotational speed, the first correspondence between absolute values of first current differences and first pump blood flow values at the rotational speed is obtained on the basis of the absolute values of the first current differences and the third correspondence. In this way, the first correspondence between the absolute values of the first current differences and the first pump blood flow values at the different rotational speed can be accurately obtained, so as to obtain accurate pump blood flow values on the basis of the accurate first correspondence.

Then, step S 120 of obtaining first reference current values of current cycles during operation of the blood pump at different rotational speeds in an actual human body environment of a test object is introduced.

The test object may be an object tested using the blood pump, which may be, for example, an object that places the blood pump in a body of an object.

The current cycles may be cycles of a current.

In some embodiments of the present application, a human heart has a cardiac cycle. Correspondingly, after the blood pump is placed in the human body, as the human heart beats periodically, the current of the blood pump also has a cycle. FIG. 6 shows a curve of current changes in a cardiac cycle at a rotational speed. In each current cycle, the current changes regularly according to the curve shown in FIG. 6.

The first reference current value may be a reference current value in each current cycle during operation of the blood pump at each rotational speed in an actual human body environment of a test object.

In some embodiments of the present application, in order to further accurately determine the pump blood flow values of the blood pump, S 120 may specifically include:
obtaining first current values of each current cycle during operation of the blood pump at each rotational speed; and
for each current cycle, determining a maximum first current value in the current cycle to be the first reference current value of the current cycle.

The first current values may be current values in each current cycle during operation of the blood pump at each rotational speed, that is, the current values as shown in FIG. 6.

In some embodiments of the present application, in a cardiac cycle, aortic and left ventricular pressures may be approximately the same when an aortic valve is open. In this case, the pressure difference between the suction end and the outflow end of the pump blood catheter of the blood pump is approximately 0. In this case, the pump blood flow is maximum, the work done by the motor is maximum, and the current is maximum. Therefore, the maximum first current value in each current cycle may be determined to be a reference current value of the current cycle (i.e., the first reference current value).

In this embodiment of the present application, first current values of each current cycle during operation of the blood pump at each rotational speed is obtained; and for each current cycle, a maximum first current value in the current cycle is determined to be the first reference current value of the current cycle. In this way, the absolute value of the second current difference can be accurately determined on the basis of the first reference current value, so as to obtain an accurate target pump blood flow value.

In some embodiments of the present application, after the blood pump is placed in the human body, if the blood pump is placed at a correct position, the current during the operation of the blood pump may change periodically. The first reference current value of each current cycle can be obtained only when the current during the operation of the blood pump changes periodically. Therefore, prior to step S120, there is a need to judge whether the current during the operation of the blood pump changes periodically. Therefore, prior to step S120, the above pump blood flow determination method for a blood pump may further include:
determining a current cycle of the blood pump at each rotational speed on the basis of a cardiac cycle of the test object at the rotational speed;
for each rotational speed, comparing the current cycle at the rotational speed with a predicted current cycle corresponding to the cardiac cycle; and
determining, on the basis of a comparison result, whether current values at the different rotational speeds change periodically.

For each rotational speed, the predicted current cycle may be a current cycle predicted by a preset algorithm for the cardiac cycle at the rotational speed.

In some embodiments of the present application, a cardiac cycle of the test object at each rotational speed may be obtained, a current cycle of the blood pump at each rotational speed may be obtained by measurement on the basis of the cardiac cycle, the current cycle is compared with the predicted current cycle, if a comparison result is that they are consistent, it is determined that the current values at the different rotational speeds change periodically, and if the comparison result is that they are inconsistent, it indicates that the blood pump is placed at an incorrect position and the blood pump is required to be repositioned.

Correspondingly, S120 may specifically be: obtaining the first reference current value of each current cycle during operation of the blood pump at each rotational speed in the actual human body environment of the test object when it is determined that the current values at the different rotational speeds change periodically.

In some embodiments of the present application, if long-term current data of the blood pump is abnormal, that is, if there is no periodic change, alarm information may be generated to prompt that the blood pump is not placed at the correct position and the blood pump is required to be repositioned.

In this embodiment of the present application, a cardiac cycle of the test object at each rotational speed is obtained, a current cycle of the blood pump at each rotational speed may be obtained by measurement on the basis of the cardiac cycle, the current cycle is compared with the predicted current cycle, and it is determined, on the basis of a comparison result, whether current values at the different rotational speeds change periodically. In this way, normal operation of the blood pump can be ensured.

Then, step S130 of for each current cycle at each rotational speed, calculating differences between the first reference current value and current values at different moments within the current cycle, so as to obtain an absolute value of at least one second current difference is introduced.

The absolute values of the second current differences may be values obtained by calculating, for each current cycle at each rotational speed, absolute values of differences between the first reference current value in the current cycle and current values at different moments in the current cycle.

In an example, for a current cycle at the rotational speed n1, a maximum current value I1 in the current cycle is obtained, the maximum current value I1 is determined to be the first reference current value, then differences between I1 and current values at different moments in the current cycle are calculated, absolute values of the differences are calculated, and absolute values of a plurality of second current differences corresponding to the current cycle at the rotational speed n1 can be obtained.

Then, step S140 of for the absolute value of the at least one second current difference corresponding to each current cycle at each rotational speed, searching in the first correspondence to obtain at least one second pump blood flow value corresponding to the absolute value of the at least one second current difference is introduced.

The second pump blood flow value may be a pump blood flow value corresponding to the absolute value of the second current difference found in the first correspondence.

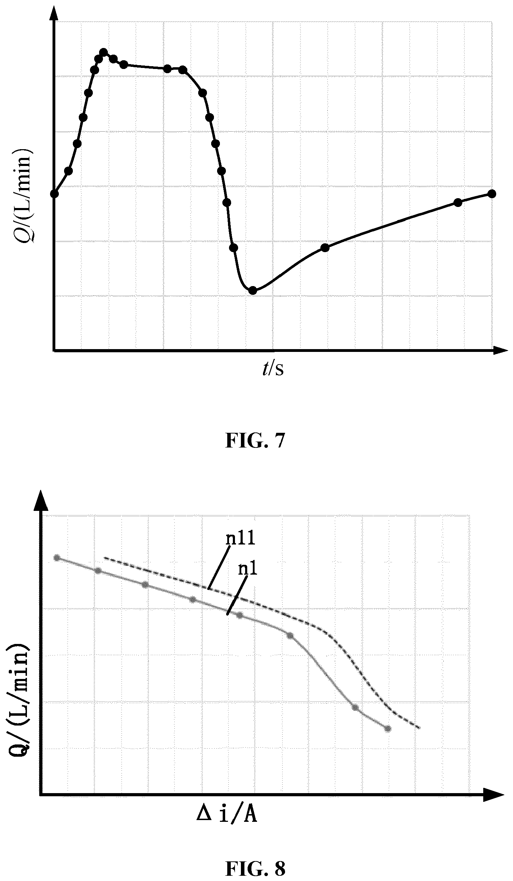

In some embodiments of the present application, since the first correspondence includes the correspondence between the absolute values of the first current differences and the first pump blood flow values at the different rotational speeds (i.e., FIG. 5), after the absolute values of the second current differences in each current cycle at each rotational speed are obtained, for example, after the absolute values of the second current differences in a current cycle at the rotational speed n1 are obtained, pump blood flow values corresponding to the absolute values of the second current differences may be searched in the first correspondence (i.e., FIG. 5), and the found pump blood flow values corresponding to the absolute values of the second current differences are determined to be the second pump blood flow values in the current cycle at the rotational speed n1. A curve of second pump blood flow values in a current cycle at a rotational speed shown in FIG. 7 may be obtained.

In some embodiments of the present application, in order to ensure accuracy of searching of the absolute values of the second current differences in FIG. 5, subsequent to step S120, the above pump blood flow determination method for a blood pump may further include:
calibrating the first reference current value to the second reference current value.

In some embodiments of the present application, after the first reference current value is determined, the first reference current value may be calibrated to the second reference current value, that is, I₁=I₀.

In some embodiments of the present application, after the second reference current value is determined, a flow value corresponding to the second reference current value may be taken as a maximum flow value Qmax in the current cycle. When the target pump blood flow value is subsequently displayed, the maximum flow value Qmax may also be displayed.

Correspondingly, step S130 may specifically include:
for each current cycle at each rotational speed, calculating differences between the second reference current value and current values at different moments within the current cycle, so as to obtain the absolute value of the at least one second current difference.

In some embodiments of the present application, after the first reference current value is calibrated to the second reference current value, for each current cycle at each rotational speed, differences between the second reference current value and current values at different moments within the current cycle may be calculated, so as to obtain the absolute value of the at least one second current difference.

In some embodiments of the present application, for each current cycle at each rotational speed, after the absolute values of the second current differences in the current cycle are obtained, a pump blood flow value corresponding to the absolute value of a maximum second current difference in the absolute values of the second current differences may be determined to be a minimum flow value Qmin in the current cycle. When the target pump blood flow value is subsequently displayed, the minimum flow value Qmin may also be displayed.

In this embodiment of the present application, the first reference current value is calibrated to the second reference current value. A same reference current value is selected in such a manner that obtained absolute values of differences (absolute values of first differences and absolute values of second differences) are obtained on the basis of the same reference current value, which ensures that, during subsequent searching in FIG. 5, pump blood flow values corresponding to the absolute values of differences obtained on the basis of the same reference current value can be obtained, so as to obtain accurate pump blood flow values.

Finally, step S150 of for each rotational speed, determining a target pump blood flow value on the basis of at least one second pump blood flow value in each current cycle is introduced.

The target pump blood flow value may be a pump blood flow value at each rotational speed obtained for the rotational speed, that is, a flow value used to represent a pump blood capability of the blood pump.

In some embodiments of the present application, in order to accurately determine the target pump blood flow value, S 150 may specifically include:
for each current cycle at each rotational speed, performing integral calculation on the at least one second pump blood flow value in the current cycle, so as to obtain a first total pump blood flow value in the current cycle;
for each current cycle at each rotational speed, determining an average pump blood flow value in the current cycle on the basis of the first total pump blood flow value and a current cycle value;
for each rotational speed, summing the average pump blood flow values in the current cycles, so as to obtain a second total pump blood flow value at the rotational speed; and
for each rotational speed, calculating an average value of the second total pump blood flow value, so as to obtain the target pump blood flow value at the rotational speed.

For each current cycle at each rotational speed, the first total pump blood flow value may be a value obtained by performing integral calculation on the at least one second pump blood flow value in the current cycle.

The current cycle value may be how long each current cycle is. For example, if a current cycle is 10 s, the current cycle value is 10 s.

For each current cycle at each rotational speed, the average pump blood flow value may be a value obtained by averaging the first total pump blood flow value in the current cycle.

For each rotational speed, the second total pump blood flow value may be a value obtained by summing average pump blood flow values in current cycles at the rotational speed.

In an example, referring to FIG. 7 which is a curve of second pump blood flow values in a current cycle at a rotational speed n1, integral calculation is performed on the second pump blood flow values in FIG. 7 to obtain a first total pump blood flow value in the current cycle, and then an average pump blood flow value in the current cycle may be obtained by dividing the first total pump blood flow value by a current cycle value. A second total pump blood flow value at the rotational speed n1 may be obtained by summing average pump blood flow values in current cycles at the rotational speed n1. A target pump blood flow value at the rotational speed may be obtained by averaging the second total pump blood flow value at the rotational speed n1.

In some embodiments of the present application, a curve of pump blood flow values in one current cycle may be obtained by fitting the above correspondence between the absolute values of the second current differences and the second pump blood flow values. The fitting may include polynomial fitting, trigonometric function fitting, exponential function fitting, logarithmic function fitting, or combinations thereof.

In this embodiment of the present application, the determined target pump blood flow value can help a clinician determine how much pump blood flow the blood pump should provide for the test object and when the blood pump can be withdrawn.

In some embodiments of the present application, after the target pump blood flow value at each rotational speed is obtained, a cardiac output value at the rotational speed may be determined on the basis of the target pump blood flow value at the rotational speed.

In some embodiments of the present application, in order to accurately determine a recovery function of the test object, subsequent to step S150, the pump blood flow determination method for a blood pump may further include:
for each rotational speed, determining a cardiac output value at the rotational speed on the basis of the target pump blood flow value at the rotational speed.

The cardiac output value may be a value used to represent a heart recovery function of the test object.

In some embodiments of the present application, the for each rotational speed, determining a cardiac output value at the rotational speed on the basis of the target pump blood flow value at the rotational speed may specifically be implemented by:
for each rotational speed, determining vascular resistance of the test object at an initial moment on the basis of a total cardiac output value of the test object at the initial moment and an average aortic pressure value of the test object at the initial moment;
for each rotational speed, determining total cardiac output values of the test object at different moments on the basis of average aortic pressure values of the test object at the different moments and the vascular resistance of the test object at the initial moment; and
for each rotational speed, determining the cardiac output value of the test object at the rotational speed on the basis of the total cardiac output values of the test object at the different moments and the target pump blood flow value.

The initial moment may be a moment when the blood pump is placed in the body of the test object and the blood pump starts operating.

In some embodiments of the present application, the total cardiac output values of the test object at the different moments may be measured and obtained by an external device, which may be obtained by, for example, a pulse indicator continuous cardiac output (PiCCO) monitoring device or a float catheter Swan - Ganz. The average aortic pressure values may alternatively be obtained by an auxiliary pressure sensor.

In some embodiments of the present application, how to specifically obtain the total cardiac output values and the average aortic pressure values of the test object at the different moments is not limited in the present application.

In some embodiments of the present application, based on the total cardiac output value and the average aortic pressure value of the test object at the initial moment, the vascular resistance of the test object at the initial moment may specifically be determined according to the following formula (1):

CO=MAP/SVR (1)

where CO denotes the total cardiac output value, MAP denotes the average aortic pressure value, and SVR denotes the vascular resistance.

The vascular resistance SVR0 of the test object at the initial moment may be calculated according to the above formula (1).

In some embodiments of the present application, since the vascular resistance remains constant over a period of time (e.g., 8 h), within the period of time, the total cardiac output value of the test object at a moment Ti may be obtained on the basis of the following formula (2):

COi=MAPTi/SVR0 (2)

Real-time COi of the test object may be calculated using the above formula (2).

For each rotational speed, the cardiac output value of the test object at the rotational speed may be determined on the basis of the total cardiac output values of the test object at the different moments and the target pump blood flow value at the rotational speed by using the following formula (3) and formula (4):

COi=Con+COC (3)

where Con denotes a cardiac output value of the test object at a rotational speed, and COC denotes a pump blood flow value of the blood pump at the rotational speed. The following formula (4) may be obtained according to the above formula (3):

Con =COi-COC (4)

Through the above formula (4), a CO value (i.e., the cardiac output value) of the test object at a rotational speed may be obtained.

In this embodiment of the present application, for each rotational speed, the cardiac output value at the rotational speed is determined on the basis of the target pump blood flow value at the rotational speed. In this way, an accurate cardiac output value can be determined on the basis of an accurate target pump blood flow value, and then a degree of recovery of the heart of the test object can be accurately known.

In some embodiments of the present application, the target pump blood flow value determined above may be obtained when the catheter of the blood pump is in a straight state, or obtained when the catheter of the blood pump is in a bending state. During actual application of the blood pump, the state of the catheter of the blood pump may be different from that in the in vitro test simulation environment. As a result, the current of the blood pump may be different in actual application, that is, the current values are abnormal, and absolute values of the current differences obtained may also be different. In this way, the first correspondence in the in vitro test simulation environment cannot be used. However, if the catheter of the blood pump does not change much during actual application compared with the state in the in vitro test simulation environment, the current values may not change much, and the target pump blood flow value may be determined on the basis of the first correspondence in the in vitro test simulation environment. Therefore, during actual application, a certain adjustment range is allowed for the absolute values of the first current differences in the in vitro test simulation environment.

In some embodiments of the present application, the catheter of the blood pump has two states: a first state and a second state.

In some embodiments of the present application, the first state may be that the catheter of the blood pump is in the straight state, and the second state may be that the catheter of the blood pump is in the bending state.

When the catheter of the blood pump is in different states, correspondingly, in the in vitro test simulation environment, obtaining, for each rotational speed, second current values under pressure differences at the rotational speed may be obtaining, for each rotational speed, second current values under pressure differences at the rotational speed in different states. The second current values herein may include first sub-current values and second sub-current values. The first sub-current values may be current values of the blood pump under the different pressure differences at the different rotational speeds when the catheter of the blood pump is in the first state. The second sub-current values may be current values of the blood pump under the different pressure differences at the different rotational speeds when the catheter of the blood pump is in the second state.

In some embodiments of the present application, in order to achieve a certain degree of correction to the absolute value of the first current difference, in S110, after the absolute values of the first current differences of the blood pump under the different pressure differences at the different rotational speeds are respectively obtained in the first state and the absolute values of the first current differences of the blood pump under the different pressure differences at the different rotational speeds are respectively obtained in the second state, the above pump blood flow determination method for a blood pump may further include:

for each pressure difference at each rotational speed, calculating a difference between the first sub-current value and the second sub-current value under the pressure difference, so as to obtain an absolute value of a third current difference; and

for each pressure difference at each rotational speed, correcting the absolute value of the first current difference in a target state on the basis of the absolute value of the third current difference.

For each rotational speed, the absolute value of the third current difference may be a value obtained by calculating an absolute value of a difference between the first sub-current value and the second sub-current value under each pressure difference at the rotational speed.

The target state may be the first state or the second state.

In some embodiments of the present application, the current values (i.e., the first sub-current values and the second sub-current values) of the blood pump under the pressure differences at the different rotational speeds in the first state and the second state may be obtained respectively, then differences between the current values under the pressure differences in the two states are calculated to obtain absolute values of third current differences, and the absolute values of the first current differences in the first state (or the second state) may be corrected by using the absolute values of the third current differences. That is, the absolute values of the current differences in the straight state may be corrected by using the absolute values of the current differences in the bending state, or the absolute values of the current differences in the bending state may be corrected by using the absolute values of the current differences in the straight state.

In an example, description is based on an example in which the absolute values of the current differences in the straight state are corrected by using the absolute values of the current differences in the bending state. At the rotational speed n1, when the catheter of the blood pump is in the straight state, the current values (i.e., the first sub-current values) under different pressure differences are 1, 2, 1, and 3 respectively. When the catheter of the blood pump is in the bending state, the current values (i.e., the second sub-current values) under different pressure differences are 1.2, 1.8, 1.1, and 3.1 respectively. Then, differences between the first sub-current values and the second sub-current values under corresponding pressure differences at the rotational speed n1 are calculated, and then absolute values of the differences are calculated, so as to obtain absolute values of third current differences, that is, |1 - 1.21=0.2, |2 - 1.81=0.2, |1 - 1.11=0.1, and |3 - 3.11=0.1. 0.2, 0.2, 0.1, and 0.1 herein are the absolute values of the third current differences.

In some embodiments of the present application, a maximum value in the absolute values of the third current differences may be taken as a correction range of the absolute values of the first current differences.

Still referring to the above example, after 0.2, 0.2, 0.1, and 0.1 are determined to be the absolute values of the third current differences, the maximum value 0.2 in 0.2, 0.2, 0.1, and 0.1 may be taken as the correction range of the absolute values of the first current differences. That is, the absolute values of the first current differences may fluctuate within a range of 0.2.

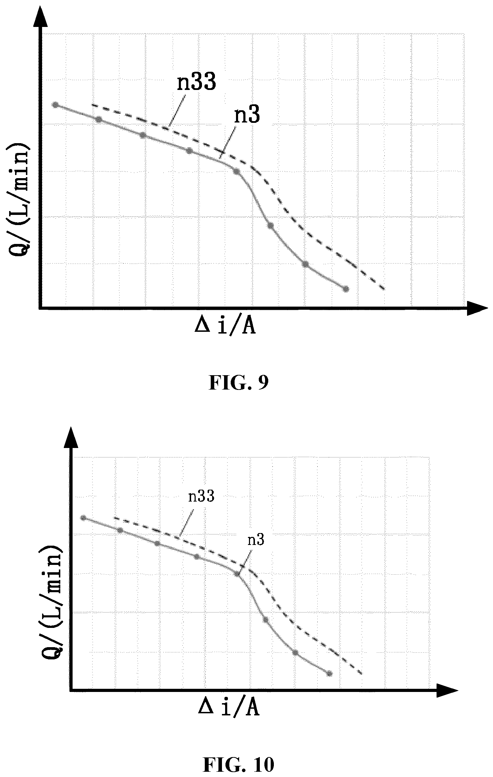

In an example, as shown in FIG. 8, a curve corresponding to n1 in FIG. 8 is a correspondence between absolute values of first current differences and first pump blood flow values at the rotational speed n1 when the catheter of the blood pump is in the straight state, and a curve corresponding to n11 in FIG. 8 is a curve formed by maximum values of the correction ranges of the absolute values of the first current differences at the rotational speed n1. Correspondingly, in FIG. 9, a curve corresponding to n2 in FIG. 9 is a correspondence between absolute values of first current differences and first pump blood flow values at the rotational speed n2 when the catheter of the blood pump is in the straight state, and a curve corresponding to n22 in FIG. 9 is a curve formed by maximum values of the correction ranges of the absolute values of the first current differences at the rotational speed n2. A curve corresponding to n3 in FIG. 10 is a correspondence between absolute values of first current differences and first pump blood flow values at the rotational speed n3 when the catheter of the blood pump is in the straight state, and a curve corresponding to n33 in FIG. 10 is a curve formed by maximum values of the correction ranges of the absolute values of the first current differences at the rotational speed n3.

In some embodiments of the present application, absolute values of current differences in one bending state may be used to correct absolute values of current differences in another bending state. For example, absolute values of current differences at a curvature of 5° may be used to correct absolute values of current differences at a curvature of 8°.

In some embodiments of the present application, if a current increment exceeds a predetermined limit (that is, exceeds the correction range of the absolute values of the first current differences) due to changes in a bending degree of the catheter or other circumstances, data of the current cycle may be discarded. After the current cycle is stable, counting is performed again to calculate the target pump blood flow value.

In this embodiment of the present application, when the blood pump is in the in vitro test simulation environment, after the first sub-current values of the blood pump under the different pressure differences at the different rotational speeds in the first state and the second sub-current values of the blood pump under the different pressure differences at the different rotational speeds in the second state are respectively obtained, for each pressure difference at each rotational speed, a difference between the first sub-current value and the second sub-current value under the pressure difference is calculated, so as to obtain an absolute value of a third current difference; and the absolute value of the first current difference in a target state are corrected on the basis of the absolute value of the third current difference. In this way, the absolute values of the first current differences are corrected, ensuring that in the actual application of the catheter of the blood pump, as long as the state thereof is within the correction range compared with the in vitro test simulation environment, the second pump blood flow value can be found directly on the basis of the first correspondence, ensuring efficiency of determination of the target pump blood flow value, reducing a value range of the correction, and improving value accuracy of the target pump blood flow value.

In some embodiments of the present application, after the target pump blood flow value is determined, the target pump blood flow value may be displayed, and at the same time, any figure in FIG. 2 to FIG. 10 and each figure in FIG. 2 to FIG. 10 may also be displayed, so that the target pump blood flow value can be viewed in a timely and intuitive manner, improving user experience.

It is to be noted that the pump blood flow determination method for a blood pump provided in the embodiments of the present application may be performed by a pump blood flow determination apparatus for a blood pump or by a control module configured to perform the pump blood flow determination method for a blood pump in the pump blood flow determination apparatus for a blood pump. In the embodiments of the present application, a pump blood flow determination apparatus for a blood pump provided in the embodiments of the present application is described based on an example in which the pump blood flow determination apparatus for a blood pump performs the pump blood flow determination method for a blood pump.

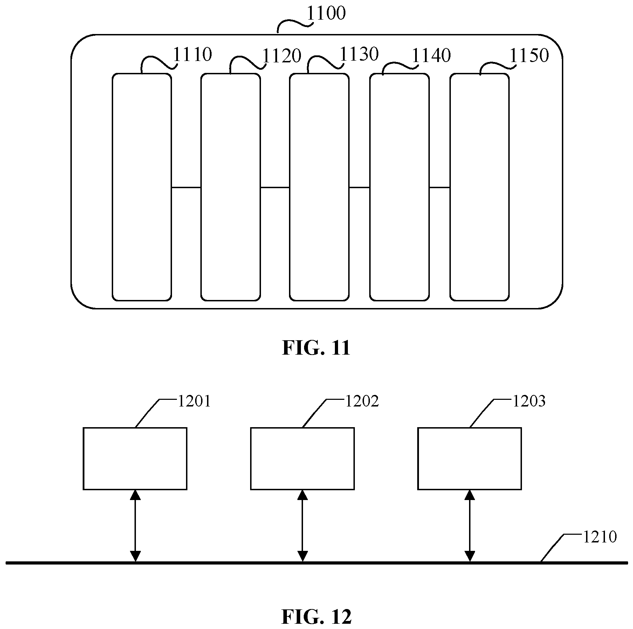

On the basis of a same inventive concept as the above pump blood flow determination method for a blood pump, the present application further provides a pump blood flow determination apparatus for a blood pump. The pump blood flow determination apparatus for a blood pump provided in an embodiment of the present application will be described in detail below with reference to FIG. 11.

FIG. 11 is a structural block diagram of a pump blood flow determination apparatus for a blood pump according to an exemplary embodiment.

As shown in FIG. 11, the pump blood flow determination apparatus for a blood pump 1100 may include:
a first obtaining module 1110 configured to obtain a first correspondence between absolute values of first current differences and first pump blood flow values at different rotational speeds of the blood pump in an in vitro test simulation environment;
a second obtaining module 1120 configured to obtain first reference current values of current cycles during operation of the blood pump at different rotational speeds in an actual human body environment of a test object;
a first determination module 1130 configured to, for each current cycle at each rotational speed, calculate differences between the first reference current value and current values at different moments within the current cycle, so as to obtain an absolute value of at least one second current difference;
a second determination module 1140 configured to, for the absolute value of the at least one second current difference corresponding to each current cycle at each rotational speed, search in the first correspondence to obtain at least one second pump blood flow value corresponding to the absolute value of the at least one second current difference; and
a third determination module 1150 configured to, for each rotational speed, determine a target pump blood flow value on the basis of at least one second pump blood flow value in each current cycle.

In this embodiment of the present application, a first correspondence between absolute values of first current differences and first pump blood flow values at different rotational speeds of the blood pump in an in vitro test simulation environment is obtained through a first obtaining module; on the basis of a second obtaining module, first reference current values of current cycles during operation of the blood pump at different rotational speeds in an actual environment of a test object are obtained; on the basis of a first determination module, for each current cycle at each rotational speed, differences between the first reference current value and current values at different moments within the current cycle are calculated, so as to obtain an absolute value of at least one second current difference; on the basis of a second determination module, for the absolute value of the at least one second current difference corresponding to each current cycle at each rotational speed, searching is performed in the first correspondence to obtain at least one second pump blood flow value corresponding to the absolute value of the at least one second current difference; and on the basis of a third determination module, for each rotational speed, a target pump blood flow value is determined on the basis of at least one second pump blood flow value in each current cycle. In this way, compared with the manner of directly using currents to represent the pump blood flow values of the blood pump, in the manner of effectively representing the pump blood flow values of the blood pump with current change differences, by using the current differences, errors caused by differences between different blood pump catheters can be effectively reduced, thereby achieving an effect of accurately determining pump blood flow of the blood pump.

In some embodiments of the present application, in order to further accurately determine the pump blood flow values of the blood pump, the second obtaining module 1120 may include:
a first obtaining unit configured to obtain first current values of each current cycle during operation of the blood pump at each rotational speed; and
a first determination unit configured to, for each current cycle, determine a maximum first current value in the current cycle to be the first reference current value of the current cycle.

In some embodiments of the present application, in order to further accurately determine the target pump blood flow value, the third determination module 1150 may include:
a second determination unit configured to, for each current cycle at each rotational speed, perform integral calculation on the at least one second pump blood flow value in the current cycle, so as to obtain a first total pump blood flow value in the current cycle;
a third determination unit configured to, for each current cycle at each rotational speed, determine an average pump blood flow value in the current cycle on the basis of the first total pump blood flow value and a current cycle value;
a fourth determination unit configured to, for each rotational speed, sum the average pump blood flow values in the current cycles, so as to obtain a second total pump blood flow value at the rotational speed; and
a fifth determination unit configured to, for each rotational speed, calculate an average value of the second total pump blood flow value, so as to obtain the target pump blood flow value at the rotational speed.

In some embodiments of the present application, in order to accurately determine a recovery function of the test object, the pump blood flow determination apparatus for a blood pump may further include:
a fourth determination module configured to, for each rotational speed, determining a cardiac output value at the rotational speed on the basis of the target pump blood flow value at the rotational speed.

In some embodiments of the present application, the fourth determination module may specifically be configured to:
for each rotational speed, determine vascular resistance of the test object at an initial moment on the basis of a total cardiac output value of the test object at the initial moment and an average aortic pressure value of the test object at the initial moment;
for each rotational speed, determine total cardiac output values of the test object at different moments on the basis of average aortic pressure values of the test object at the different moments and the vascular resistance of the test object at the initial moment; and
for each rotational speed, determine the cardiac output value of the test object at the rotational speed on the basis of the total cardiac output values of the test object at the different moments and the target pump blood flow value.

In some embodiments of the present application, in order to further accurately determine the target pump blood flow value of the blood pump, the pump blood flow determination apparatus for a blood pump may further include:
a third obtaining module configured to obtain a second correspondence between pressure differences and second current values and a third correspondence between the pressure differences and the first pump blood flow values of the blood pump at the different rotational speeds when the blood pump is in the in vitro test simulation environment;
a fifth determination module configured to, for each rotational speed, take the corresponding second current value under a zero pressure difference as a second reference current value;
a sixth determination module configured to, for each rotational speed, obtain second current values under different pressure differences at the rotational speed on the basis of the second correspondence, and calculate differences between the second reference current value and second current values under the different pressure differences at the rotational speed, so as to obtain absolute values of first current differences under the different pressure differences; and
a seventh determination module configured to, for each rotational speed, obtain the first correspondence between absolute values of first current differences and first pump blood flow values at the rotational speed on the basis of the absolute values of the first current differences and the third correspondence.

In some embodiments of the present application, in order to further accurately determine the target pump blood flow value of the blood pump, the pump blood flow determination apparatus for a blood pump may further include:
an eighth determination module configured to calibrate the first reference current value as the second reference current value.

Correspondingly, the first determination module 1130 may specifically be configured to:
for each current cycle at each rotational speed, calculate differences between the second reference current value and current values at different moments within the current cycle, so as to obtain the absolute value of the at least one second current difference.

In some embodiments of the present application, in order to further accurately determine the target pump blood flow value of the blood pump, when the blood pump is in the in vitro test simulation environment, states of a catheter of the blood pump include a first state and a second state; and the second current values include first sub-current values and second sub-current values, wherein the first sub-current values are current values of the blood pump under the different pressure differences at the different rotational speeds when the catheter of the blood pump is in the first state, and the second sub-current values are current values of the blood pump under the different pressure differences at the different rotational speeds when the catheter of the blood pump is in the second state.

The pump blood flow determination apparatus for a blood pump may further include:
a correction module configured to, for each pressure difference at each rotational speed, calculate a difference between the first sub-current value and the second sub-current value under the pressure difference, so as to obtain an absolute value of a third current difference; and for each pressure difference at each rotational speed, correct the absolute value of the first current difference in a target state on the basis of the absolute value of the third current difference; wherein the target state is the first state or the second state.

In some embodiments of the present application, in order to determine that the current during the operation of the blood pump changes periodically, the pump blood flow determination apparatus for a blood pump may further include:
a tenth determination module configured to determine a current cycle of the blood pump at each rotational speed on the basis of a cardiac cycle of the test object at the rotational speed;
a comparison module configured to, for each rotational speed, compare the current cycle at the rotational speed with a predicted current cycle corresponding to the cardiac cycle; and
a comparison and determination module configured to determine, on the basis of a comparison result, whether current values at the different rotational speeds change periodically.

Correspondingly, the second obtaining module 1120 may specifically be configured to:
obtain the first reference current value of each current cycle during operation of the blood pump at each rotational speed in the actual human body environment of the test object when it is determined that the current values at the different rotational speeds change periodically.

The pump blood flow determination apparatus for a blood pump provided in this embodiment of the present application may be configured to perform the pump blood flow determination method for a blood pump provided in the above method embodiments, and implementation principles and technical effects thereof are similar. Details are not described herein again for the sake of simplicity.

Based on a same inventive concept, an embodiment of the present application further provides an electronic device.

FIG. 12 is a schematic structural diagram of an electronic device according to an embodiment of the present application. As shown in FIG. 12, the electronic device may include a processor 1201 and a memory 1202 storing computer programs or instructions.

Specifically, the processor 1201 may include a central processing unit (CPU), or an application specific integrated circuit (ASIC), or may be configured to implement one or more integrated circuits according to the embodiments of the present application.

The memory 1202 may include a mass memory for data or instructions. By way of example instead of limitation, the memory 1202 may include a hard disk drive (HDD), a floppy disk drive, a flash memory, an optical disk, a magneto-optical disk, a magnetic tape or a universal serial bus (USB) drive, or a combination of two or more of these. Where appropriate, the memory 1202 may include a removable or non-removable (or fixed) medium. Where appropriate, the memory 1202 may be internal or external to an integrated gateway disaster recovery device. In a particular embodiment, the memory 1202 is a non-volatile solid-state memory. In a particular embodiment, the memory 1202 includes a read-only memory (ROM). Where appropriate, the ROM may be a mask-programmed ROM, a programmable ROM (PROM), an erasable PROM (EPROM), an electrically erasable PROM (EEPROM), an electrically rewritable ROM (EAROM) or a flash memory, or a combination of two or more of these.

The processor 1201 reads and executes the computer programs or instructions stored in the memory 1202 to implement the pump blood flow determination method for a blood pump in any of the above embodiments.

In an example, the electronic device may further include a communication interface 1203 and a bus 1210. As shown in FIG. 12, the processor 1201, the memory 1202, and the communication interface 1203 are connected to each other through the bus 1210 and complete communication with each other.

The communication interface 1203 is mainly used to implement communication between various modules, devices, units, and/or devices in the embodiments of the present application.

The bus 1210 includes hardware, software, or both, and couples components of the electronic device to each other. By way of example instead of limitation, the bus may include an accelerated graphics port (AGP) or other graphics buses, an enhanced industry standard architecture (EISA) bus, a front side bus (FSB), a hypertransport (HT) interconnect, an industry standard architecture (ISA) Bus, an unlimited bandwidth interconnect, a low pin count (LPC) bus, a memory bus, a microchannel architecture (MCA) bus, a peripheral component interconnect (PCI) bus, a PCI-Express (PCI-X) bus, a serial advanced technology attachment (SATA) bus, a video electronics standards association local bus (VLB), or other suitable buses, or a combination of two or more of these. Where appropriate, the bus 1210 may include one or more buses. Although specific buses are described and illustrated in the embodiments of the present application, the present application contemplates any suitable bus or interconnect.

The electronic device may perform the pump blood flow determination method for a blood pump in the embodiments of the present application, so as to implement the pump blood flow determination method for a blood pump described in any of FIG. 1 to FIG. 10.

In addition, with reference to the pump blood flow determination method for a blood pump in the above embodiments, an embodiment of the present application may provide a readable storage medium for implementation. The readable storage medium stores program instructions. The program instructions, when executed by a processor, implement the pump blood flow determination method for a blood pump described in any of the above embodiments.

It should be clear that the present application is not limited to the specific configuration and processing described above and shown in the figures. For the sake of brevity, a detailed description of any known method has been omitted herein. In the above embodiments, several specific steps are described and shown as examples. However, the method process of the present application is not limited to the specific steps described and shown. After understanding the spirit of the present application, those skilled in the art can make various changes, modifications, and additions, or change the sequence between the steps.

The functional blocks shown in the structural block diagram described above may be implemented in hardware, software, firmware, or a combination thereof. When implemented in hardware, the functional blocks may be, for example, an electronic circuit, an ASIC, appropriate firmware, a plug-in, a function card, and the like. When implemented in software, elements of the present application are programs or code segments used to perform required tasks. The programs or code segments may be stored in a machine-readable medium or transmitted over a transmission medium or a communication link via data signals carried in carriers. The "machine-readable medium" may include any medium capable of storing or transmitting information. Examples of the machine-readable medium include an electronic circuit, a semiconductor memory device, a ROM, a flash memory, an erasable ROM (EROM), a floppy disk, a CD-ROM, an optical disk, a hard disk, a fiber medium, a radio frequency (RF) link, and the like. The code segments may be downloaded via a computer network, such as the Internet, an intranet, or the like.

It should also be noted that the exemplary embodiments mentioned in the present application describe some methods or systems based on a series of steps or apparatuses. However, the present application is not limited to the order of the above steps. That is, the steps may be performed in the order mentioned in the embodiments, or may be performed in a different order than the embodiments, or several steps may be performed simultaneously.

Those skilled in the art will appreciate that the above embodiments are illustrative and not restrictive. Different technical features that appear in different embodiments can be combined to achieve a beneficial effect. Those skilled in the art can understand and implement other modified embodiments of the disclosed embodiments on the basis of studying the drawings, the specification, and the claims. In the claims, the term "comprise" does not exclude other apparatuses or steps; an item not modified with a quantifier is intended to include one or more items, and may be used interchangeably with "one or more items"; and the terms "first" and "second" are used to label items rather than illustrate any particular order. Any reference sign in the claims should not be construed as limiting the protection scope. Functions of several parts in the claims may be implemented by a single hardware or software module. Existence of certain technical features in different dependent claims does not mean that these technical features cannot be combined to achieve a beneficial effect.

## Claims

1. A pump blood flow determination method for a blood pump, the method comprising:
obtaining a first correspondence between absolute values of first current differences and first pump blood flow values at different rotational speeds of the blood pump in an in vitro test simulation environment;
obtaining first reference current values of current cycles during operation of the blood pump at different rotational speeds in an actual environment of a test object;
for each current cycle at each rotational speed, calculating differences between the first reference current value and current values at different moments within the current cycle, so as to obtain an absolute value of at least one second current difference;
for the absolute value of the at least one second current difference corresponding to each current cycle at each rotational speed, searching in the first correspondence to obtain a second pump blood flow value corresponding to the absolute value of the second current difference, wherein the absolute value of each second current difference corresponds to a second pump blood flow value; and
for each rotational speed, determining a target pump blood flow value on the basis of at least one second pump blood flow value in each current cycle.

2. The method of claim 1, wherein the step of obtaining first reference current values of current cycles during operation of the blood pump at different rotational speeds comprises:
obtaining first current values of each current cycle during operation of the blood pump at each rotational speed; and
for each current cycle, determining a maximum first current value in the current cycle to be the first reference current value of the current cycle.

3. The method of claim 1, wherein the for each rotational speed, determining a target pump blood flow value on the basis of at least one second pump blood flow value in each current cycle comprises:
for each current cycle at each rotational speed, performing integral calculation on the at least one second pump blood flow value in the current cycle, so as to obtain a first total pump blood flow value in the current cycle;
for each current cycle at each rotational speed, determining an average pump blood flow value in the current cycle on the basis of the first total pump blood flow value and a current cycle value;
for each rotational speed, summing the average pump blood flow values in the current cycles, so as to obtain a second total pump blood flow value at the rotational speed; and
for each rotational speed, calculating an average value of the second total pump blood flow value, so as to obtain the target pump blood flow value at the rotational speed.

4. The method of any of claims 1 to 3, wherein subsequent to the for each rotational speed, determining a target pump blood flow value on the basis of at least one second pump blood flow value in each current cycle, the method further comprises:
for each rotational speed, determining a cardiac output value at the rotational speed on the basis of the target pump blood flow value at the rotational speed.

5. The method of claim 4, wherein the for each rotational speed, determining a cardiac output value at the rotational speed on the basis of the target pump blood flow value at the rotational speed comprises:
for each rotational speed, determining vascular resistance of the test object at an initial moment on the basis of a total cardiac output value of the test object at the initial moment and an average aortic pressure value of the test object at the initial moment;
for each rotational speed, determining total cardiac output values of the test object at different moments on the basis of average aortic pressure values of the test object at the different moments and the vascular resistance of the test object at the initial moment; and
for each rotational speed, determining the cardiac output value of the test object at the rotational speed on the basis of the total cardiac output values of the test object at the different moments and the target pump blood flow value.

6. The method of any of claims 1 to 3, wherein prior to the obtaining a correspondence between absolute values of first current differences and first pump blood flow values at different rotational speeds of the blood pump in an in vitro test simulation environment, the method further comprises:
obtaining a second correspondence between pressure differences and second current values and a third correspondence between the pressure differences and the first pump blood flow values of the blood pump at the different rotational speeds when the blood pump is in the in vitro test simulation environment;
for each rotational speed, taking the corresponding second current value under a zero pressure difference as a second reference current value;
for each rotational speed, obtaining second current values under different pressure differences at the rotational speed on the basis of the second correspondence, and calculating differences between the second reference current value and second current values under the different pressure differences at the rotational speed, so as to obtain absolute values of first current differences under the different pressure differences; and
for each rotational speed, obtaining the first correspondence between absolute values of first current differences and first pump blood flow values at the rotational speed on the basis of the absolute values of the first current differences and the third correspondence.

7. The method of claim 6, wherein subsequent to the step of obtaining first reference current values of current cycles during operation of the blood pump at different rotational speeds in an actual human body environment, the method further comprises:
calibrating the first reference current value to the second reference current value; and
the for each current cycle at each rotational speed, calculating differences between the first reference current value and current values at different moments within the current cycle, so as to obtain an absolute value of at least one second current difference comprises:
for each current cycle at each rotational speed, calculating differences between the second reference current value and current values at different moments within the current cycle, so as to obtain the absolute value of the at least one second current difference.

8. The method of claim 6, wherein when the blood pump is in the in vitro test simulation environment, states of a catheter of the blood pump comprise a first state and a second state; and the second current values comprise first sub-current values and second sub-current values, wherein the first sub-current values are current values of the blood pump under the different pressure differences at the different rotational speeds when the catheter of the blood pump is in the first state, and the second sub-current values are current values of the blood pump under the different pressure differences at the different rotational speeds when the catheter of the blood pump is in the second state; and
after the absolute values of the first current differences of the blood pump under the different pressure differences at the different rotational speeds are respectively obtained in the first state and the absolute values of the first current differences of the blood pump under the different pressure differences at the different rotational speeds are respectively obtained in the second state, the method further comprises:
for each pressure difference at each rotational speed, calculating a difference between the first sub-current value and the second sub-current value under the pressure difference, so as to obtain an absolute value of a third current difference; and
for each pressure difference at each rotational speed, correcting the absolute value of the first current difference in a target state on the basis of the absolute value of the third current difference;
wherein the target state is the first state or the second state.

9. The method of any of claims 1 to 3, wherein prior to the step of obtaining first reference current values of current cycles during operation of the blood pump at different rotational speeds in an actual human body environment of a test object, the method further comprises:
determining a current cycle of the blood pump at each rotational speed on the basis of a cardiac cycle of the test object at the rotational speed;
for each rotational speed, comparing the current cycle at the rotational speed with a predicted current cycle corresponding to the cardiac cycle; and
determining, on the basis of a comparison result, whether current values at the different rotational speeds change periodically; and
the obtaining first reference current values of current cycles during operation of the blood pump at different rotational speeds in an actual human body environment comprises:
obtaining the first reference current value of each current cycle during operation of the blood pump at each rotational speed in the actual human body environment of the test object when it is determined that the current values at the different rotational speeds change periodically.

10. A pump blood flow determination apparatus for a blood pump, wherein the apparatus comprises:
a first obtaining module configured to obtain a first correspondence between absolute values of first current differences and first pump blood flow values at different rotational speeds of the blood pump in an in vitro test simulation environment;
a second obtaining module configured to obtain first reference current values of current cycles during operation of the blood pump at different rotational speeds in an actual human body environment of a test object;
a first determination module configured to, for each current cycle at each rotational speed, calculate differences between the first reference current value and current values at different moments within the current cycle, so as to obtain an absolute value of at least one second current difference;
a second determination module configured to, for the absolute value of the at least one second current difference corresponding to each current cycle at each rotational speed, search in the first correspondence to obtain at least one second pump blood flow value corresponding to the absolute value of the at least one second current difference; and
a third determination module configured to, for each rotational speed, determine a target pump blood flow value on the basis of at least one second pump blood flow value in each current cycle.

11. An electronic device, comprising a processor, a memory, and programs or instructions stored in the memory and executable on the processor, wherein the programs or instructions, when executed by the processor, implement steps of the pump blood flow determination method for a blood pump of any of claims 1 to 9.

12. A readable storage medium, wherein the readable storage medium stores programs or instructions, and the programs or instructions, when executed by a processor, implement steps of the pump blood flow determination method for a blood pump of any of claims 1 to 9.
